# EUROPEAN PATENT APPLICATION

(11) **EP 4 331 540 A1**
(43) Date of publication of application: **06.03.2024**
(21) Application number: 22193651.1
(22) Date of filing: 02.09.2022
(51) Int. Cl.: A61F 2/954, A61B 18/12, A61F 2/06

(54) **SYSTEMS FOR MINIMALLY INVASIVE CREATION OF ANASTOMOSES**

(71) Applicant: PerAGraft GmbH, 52070 Aachen (DE); RWTH Aachen, 52062 Aachen (DE)
(72) Inventor: The designation of the inventor has not yet been filed
(74) Representative: Vossius & Partner Patentanwälte Rechtsanwälte mbB

(57) **Abstract**

Systems and associated methods for minimally invasive creation of anastomoses, in particular the creation of anastomoses to the central vasculature, are disclosed. The systems comprise a delivery catheter configured for percutaneous transluminal insertion through a duct of a patient's body to a target region and a stent graft arranged within the delivery catheter. The stent graft comprises a main tube and at least one branch tube. The branch tube extends from a first end to a second end, wherein the first end is coupled to the main tube.

## Description

### Background

The present invention relates to systems and methods for minimally invasive creation of anastomoses, in particular the creation of anastomoses to the central vasculature.

Cardiac insufficiencies and angiopathies, e.g. aortic aneurysms or aortic outlet stenoses, are frequent chronic diseases. Both in the industrial nations and in the developing countries they are one of the most common causes of death. Patients suffering from them are often dependent on invasive cardiothoracic and vascular interventions. Due to their critical morbidities, the major percentage of these patients are often not suitable for the needed surgical therapies.

In various cases, the therapies are linked to gaining access to the central thoracic vessels to generate large-bore vascular anastomoses for different purposes. These kinds of interventions are still an open-surgical domain. Open surgeries result in significant trauma for the body. Moreover, the risks for complications in open surgery are significantly higher than in catheter-based interventions. Scientific research has shown the advantages of minimally invasive techniques, e.g. improved patient outcomes and lowering costs in the health care system.

Various minimally invasive systems and methods have been described for cardiothoracic and vascular surgeries.

For example, EP 3 578 134 A2, EP 3 578 135 A1 and EP 3 578 136 A1 disclose stent grafts and associated methods for treating aneurysms in the thoracic aorta via a frozen elephant trunk technique. As described, for example, in EP 3 578 136 A1, the stent graft may include bridging branches to which graft extensions may be coupled.

WO 2012/063242 A1 discloses an apparatus for performing bypass graft surgery on a patient, between a major blood vessel and a vessel occluded by a lesion. The device includes a blocking balloon, an endoscopic delivery device, a distal suture structure, a proximal suture structure and a delivery catheter. The distal suture structure is positioned within the distal end of a vessel graft for suturing the vessel graft with the vessel at a position distal to the lesion. The proximal suture structure is positioned within the proximal end of the vessel graft for suturing the vessel graft with the major blood vessel.

US 6 475 226 B1 discloses devices and methods for percutaneous transluminal minimally invasive coronary surgery, particularly bypass surgery, involving the following basic steps: determining a proper location for treatment, navigating a suitable device to the treatment site, creating an extravascular opening and pathway, guiding and/or monitoring the progress of creating the opening and pathway, and maintaining the extravascular opening and pathway. One or more extravascular openings and/or pathways may be created to define a fluid path or bypass around a vascular restriction.

US 6 309 416 B1 discloses a connector for use in providing an anastomotic connection between two tubular body fluid conduits in a patient as well as apparatus for use in delivering and deploying a connector.

EP 2 210 248 B1 discloses an intraluminal bypass prosthesis that includes a first prosthetic module, a second prosthetic module, and a third prosthetic module. The first and second prosthetic modules each have a first end and a second end, and a fenestration disposed between the first and second ends. The third prosthetic module has a first end that is sealingly engageable within the first module fenestration and a second end that is sealingly engageable within the second module fenestration. The third prosthetic module has a lumen for providing fluid communication between the first and second prosthetic modules.

Despite various systems and methods being known, open surgery still remains the option of choice for many interventions. It is thus believed that further improvements in such systems and methods are still required.

The present invention aims to provide systems, devices and methods that further improve and simplify the minimally invasive creation of anastomoses, in particular the creation of large-bore anastomoses to the central vasculature.

### Summary

According to a first aspect, the present invention relates to a system for minimally invasive creation of an anastomosis to a duct in a body of a patient, the duct comprising a duct wall. The anastomosis is created in a target region of the duct at an anastomosis site where the duct wall is punctured in order to create the anastomosis. The duct may be a vessel or a blood vessel, in particular a thoracic blood vessel (such as the thoracic artery or vein).

The system comprises a delivery catheter configured for percutaneous transluminal insertion through the duct to the target region and a stent graft arranged within the delivery catheter. The stent graft comprises a main tube and at least one branch tube. The branch tube extends from a first end to a second end, wherein the first end is coupled to the main tube. The system may be a system for generation of a large-bore central vascular anastomosis.

Preferably, the branch tube is firmly coupled, sewed to, adhered to, knitted with and/or intertwined with the main tube at the first end. For example, the first end of the branch tube may be sewed to a peripheral wall of the main tube by a suture, such as a surgical suture (e.g. PROLENE, USP 4-0). Continuous or discontinuous stiches may be used. The main tube and the branch tube may be manufactured as a unitary and/or monolithic fabric, as described in WO 2020/225254 A1 (which is hereby incorporated by reference in its entirety), which may be knitted.

In other words, the first end is already attached to the main tube when the stent graft is inserted into the body via the delivery catheter. Preferably, the first end is attached to the main tube before inserting the stent graft into the delivery catheter. For example, the first end may be attached to the main tube during manufacturing of the stent graft.

Preferably, the main tube comprises a fenestration for providing a liquid connection between the main tube and the branch tube, wherein the first end is attached to the main tube around said fenestration.

The fenestration may have a diameter of at least 3, at least 4 mm mm, preferably at least 8 mm.

The delivery catheter and/or the main tube is configured to be guided to the target region via a first guidewire, the main tube being configured to be expanded in the duct. The branch tube is configured to be guided to the anastomosis site via a second guidewire, wherein the second end of the branch tube is configured for being inserted through the perforated duct wall at the anastomosis site. In other words, the first guidewire may extend through the main tube while the delivery catheter is percutaneously inserted through the duct. The second guidewire may extend through the branch tube (and, optionally, at least partially through the main tube) while the delivery catheter is percutaneously inserted through the duct.

As will be appreciated by those skilled in the art, the present invention relies on an "inside out" technique, wherein the duct is perforated by extending a puncturing device from the inside of the duct through the duct wall. The puncturing device is percutaneously extended to the anastomosis site through the duct. The puncturing device may be, for example, the second guidewire. For this purpose, the second guidewire may be provided with an electrocautery tip configured to perforate the duct wall.

As will be appreciated by those skilled in the art, the stent graft may be arranged within the delivery catheter in a compressed and/or folded insertion configuration. The main tube may be expanded to an expanded configuration in the duct, whereas the branch tube may be configured for insertion through the punctured duct wall from within the duct towards the outside of the duct. The branch tube may be at least partially inserted through the perforated duct wall before expansion of the main tube, after partial expansion of the main tube, or after full expansion of the main tube. The preferred order may vary depending on the configuration of the delivery catheter, as further described below.

The first and/or second guidewire may be part of the claimed system.

For example, the first and/or the second guidewire may form part of the delivery catheter. A distal or leading end of the first guidewire may be advanced to the target region. The delivery catheter may be advanced to said target region over the first guidewire with the second guidewire being arranged within the delivery catheter in a retracted position. Once the target region is reached, the second guidewire may be advanced to an extended position to perforate and extend through the duct wall at the anastomosis site. The second guidewire may temporarily remain extending through the duct wall, thereby guiding the branch tube through the duct wall when the branch tube is advanced along and/or moved through the duct wall together with the second guidewire. In other words, the second guidewire may be configured to be pushed along the duct to the target region together with the delivery catheter and, optionally, while being located in the branch tube. The second guidewire may be configured to remain stationary in the branch tube while the delivery catheter is advanced over the first guidewire.

Alternatively, the second guidewire may be inserted into the body before the delivery catheter. For example, the first guidewire may be advanced to the target region and the second guidewire may be advanced to the anastomosis site independently from each other. A distal end of the second guidewire may be used to perforate the duct wall. The distal end of the second guidewire may be steered via a steerable sheath in order to perforate the duct wall at the desired anastomosis site.

A proximal end of the first guidewire and/or a proximal end of the second guidewire that remain outside the body of the patient may subsequently be inserted into the delivery catheter.

The delivery catheter may be advanced simultaneously over the first and second guidewires in the distal direction, in particular when the first and second guidewires are inserted into the body before the delivery catheter. Such approach may also be referred to as a "simultaneous" approach in the context of the present disclosure.

The duct wall may be perforated similarly to the approach described in Greenbaum et al., Transcaval Access and Closure for Transcatheter Aortic Valve Replacement: A Prospective Investigation. J Am Coll Cardiol, 2017. 69(5): p. 511-521. Without wanting to be bound by theory, it is believed that the aorta's antero-lateral wall may be an example of a favorable site for performing the perforation.

The stent graft may comprise a graft and at least one stent.

The graft may be formed by the main tube and the branch tube.

The graft may be provided by a textile structure (e.g. woven, knitted, braided or non-woven). The textile structure may be made from a polymeric material. The textile structure, i.e. the main tube and/or the branch tube, may be coated on the inside and/or the outside to prevent blood from seeping through the graft. Alternatively or additionally, the textile structure may be self-sealing upon contact with blood, as known in the art.

The stent may be attached to the main tube along an inner surface of the main tube or along an outer surface of the main tube, as known in the art. In other words, the main tube may extend at least partially or entirely around the stent or the stent may extend at least partially or entirely around the main tube.

The stent graft may comprise at least one additional stent attached to the branch tube along an inner surface of the branch tube or along an outer surface of the branch tube.

The stent or stents may be self-expandable or balloon-expandable. When one or more self-expandable stents are used, the delivery catheter may comprise a holding portion in which the respective stent is arranged in the insertion configuration. The holding portion may be covered by at least one primary sheath that is movable in the proximal or distal direction to expose and release the stent graft. The primary sheath may be translated along the longitudinal axis of the delivery catheter in the proximal or distal direction with respect to the main tube in order to release the stent graft in the duct. When a balloon-expandable stent is used, the delivery catheter comprises at least one balloon for expanding the stent graft.

The at least one stent may be formed from a metallic or polymeric material.

The stent may be sewed to the stent graft, in particular to the main tube. Alternatively, the stent graft may be attached to the main tube as described in WO 2020/002371 A1 (which is hereby incorporated by reference in its entirety).

The main tube may be configured to fixate the stent graft in the duct and/or seal against the duct wall in order to avoid or minimize leakage at the anastomosis site. The main tube may be configured to abut against the inner circumferential surface of the duct along the target region.

The delivery catheter may comprise an elongate shaft extending to a handle, the handle remaining accessible outside the body of the patient during percutaneous transluminal insertion of the delivery catheter. The shaft may be an inner shaft extending through the primary sheath.

According to a first embodiment, the stent graft is arranged within the delivery catheter with the branch tube in an inverted position, wherein the second end of the branch tube is located within the main tube in said inverted position. The system may be configured for everting the branch tube from the inverted position to an everted position, wherein the second end of the branch tube is located outside the main tube in said everted position. In other words, the branch tube may extend away from the main tube in said everted position.

More specifically, the branch tube may be configured for being at least partially or entirely inserted through the duct wall by everting the branch tube from said inverted position to said everted position.

The stent graft may be generally T-shaped when the branch tube is everted.

The delivery catheter preferably is configured such that the second guidewire extends through the branch tube.

Various mechanisms are envisaged for everting the branch tube from its inverted to its everted position.

For example, the branch tube may be mechanically coupled to the second guidewire. The branch tube may then be moved from the inverted to the everted position by pushing and/or pulling the second guidewire through the punctured duct wall.

According to one exemplary implementation, the system and/or the delivery catheter may comprise a flexible wire or thread. A distal end of the flexible wire or thread may be coupled to the branch tube, e.g. at the second end of the branch tube. A proximal end of the flexible wire of thread may remain outside the body of the user when the delivery catheter is inserted into the duct. The proximal end of the flexible wire or thread may be coupled to the proximal end of the second guidewire. The proximal end of the second guidewire with the flexible wire or thread coupled thereto may then be pulled distally through the delivery catheter, the branch tube and the punctured duct wall in order to evert the branch tube. In particular, the second guidewire may be pulled all the way through and out of the delivery catheter. This may be achieved, for example, by extending the distal end of the second guidewire through the duct wall and grasping it with forceps arranged outside the duct. The forceps may be inserted into the body via a separate access site, e.g. a minimally invasive access (for example laparoscopically). The forceps may be laparoscopic forceps.

Once the branch tube has been everted and at least partially passed through the perforated duct wall by pulling the flexible wire or thread through the branch tube and the duct wall, the flexible wire or thread may be cut off. For example, the flexible wire or thread may be cut off proximate the second end of the branch tube.

The flexible wire or thread may extend proximally along the shaft and remain accessible outside the body, e.g. at the handle.

The second guidewire may comprise a hook at its proximal end. The hook may facilitate intraoperative coupling of the flexible wire or thread to the second guidewire.

In this first embodiment, the branch tube may be configured to be everted before or after the main tube has been expanded.

According to a second embodiment, the stent graft is arranged within the delivery catheter with the second end of the branch tube located outside the main tube. In other words, the stent graft may be arranged in the delivery catheter with branch tube extending from the main tube. In particular, the branch tube may extend outwardly from the main tube at the first end. To minimize the space required in the insertion configuration, the branch tube may be folded against and/or extend parallel to the main tube along the remaining length of the branch sheath.

In the second embodiment, the delivery catheter preferably comprises at least one branch sheath arranged over the branch tube. The branch sheath may be used to compress the branch tube.

The second guidewire may extend through the branch tube and/or through the branch sheath. The second guidewire may extend out of the branch sheath through a hole provided at the distal end of the branch sheath.

The second guidewire may be configured for guiding the branch sheath towards the anastomosis site and/or through the duct wall. In particular, the second guidewire or another puncturing device may be used to perforate the duct wall. The second guidewire may be extended through the duct wall. Subsequently, the branch sheath, together with the branch tube arranged therein, may be advanced along said second guidewire towards the punctured anastomosis site.

The branch sheath may be configured for guiding the branch tube into and/or through the punctured duct wall. As such, the branch sheath may be configured for being at least partially or entirely inserted through the duct wall. In particular, the branch sheath may be configured for dilating the perforation in the duct wall when being inserted therethrough. For example, the branch sheath may be provided with a shape at its distal end, wherein the shape tapers in the distal direction (e.g., a conical distal end).

The branch sheath may be stiffer than the branch tube. In particular, the branch sheath may be more resistant to kinking than the branch tube when compressed along its longitudinal axis. This facilitates insertion of the branch sheath through the duct wall.

Preferably, the branch tube is at least partially passed through the perforated duct wall before the main tube is fully expanded in the duct, more preferably while the main tube remains in its compressed insertion configuration. In this manner, the branch tube may first be positioned as required by the surgeon. Subsequently, the position may be fixated by expanding the main tube.

The branch sheath may be configured for being removed from the branch tube once the branch tube has been at least partially guided through and/or at least partially passed through the perforated duct wall. In particular, the branch sheath may be configured to be removed once the branch tube protrudes through the perforated duct wall at least partially towards the outside. The branch tube may then be grasped on the outside of the duct with forceps. The forceps may be used to hold the branch tube in place and/or to pull the branch tube further through the perforated duct wall before or after expansion of the main tube. Preferably, the forceps are used to hold the branch tube in place during expansion of the main tube. The forceps may be laparoscopically inserted through a separate access site. The forceps may be laparoscopic forceps.

For example, the branch sheath may be removed from the branch tube by pulling the branch sheath off the branch tube in the distal direction. For example, the distal end of the branch sheath may be pushed through the duct wall to an extent that the distal end can be grasped with forceps from outside the duct. The forceps may be laparoscopically inserted through a separate access site, as explained above.

The branch sheath may be pulled through the perforated duct wall completely. The branch sheath may be pulled off the remaining device. The branch sheath may then be removed from the body with the forceps.

Without wanting to be bound by theory, it is believed that such approach is particularly advantageous because it allows for a simple design of the branch sheath and minimizes trauma to the duct wall at the anastomosis site.

Alternatively, the device may be provided with a mechanism for removing the branch sheath from the branch tube by pushing it distally off the branch tube. The branch sheath may then be retracted into the duct and/or into the delivery catheter by pulling it through the expanded branch tube in an inverted configuration.

The delivery catheter of the second embodiment may comprise a primary sheath and a secondary sheath.

The primary sheath may extend around the main tube and the branch tube. The primary sheath may be configured to hold the branch tube in a folded configuration in which the branch tube is folded against the main tube and/or against the secondary sheath. The primary sheath may be configured to hold the branch tube in a folded configuration in which the branch tube extends parallel to the main tube along a substantial portion thereof. In the folded configuration, the branch tube's second end may be arranged distally of the branch tube's first end within the delivery catheter.

The secondary sheath may extend around the main tube. The secondary sheath may be configured to hold the main tube in the compressed insertion configuration.

The secondary sheath may comprise a peripheral wall and an opening extending through said peripheral wall. The branch tube may extend through said opening when branch tube is in the folded configuration. For example, the branch tube may extend through said opening when the stent graft is arranged in the delivery device in the insertion configuration and/or when the primary sheath overlaps with the stent graft. The opening may be provided by a slot extending along the peripheral wall of the secondary sheath. The opening may extend in the longitudinal direction of the delivery catheter and/or the secondary sheath.

The opening may extend along the peripheral wall up to a distal end or up to a proximal end of the secondary sheath. In this manner, the secondary sheath may be removed from the main tube by pulling the secondary sheath in the proximal direction or by pushing the secondary sheath in the distal direction, respectively, despite the branch tube extending through the opening.

A length of the opening in the longitudinal direction of the secondary sheath may be less than 7 cm, preferably less than 5 cm. The length of the opening in the longitudinal direction of the secondary sheath may be at least 1 cm or at least 2 cm, preferably at least 4 cm.

In the second embodiment, the stent graft may be implanted by (i) advancing the distal end of the first guidewire to the target region, (ii) perforating the duct wall at the anastomosis site with the second guidewire (as described above, the second guidewire may be inserted before advancing the delivery catheter to the target region over the first guidewire or the second guidewire may be advanced to the target region together with the delivery catheter), (iii) removing the primary sheath from over the stent graft (e.g., by moving the primary sheath in the proximal or distal direction), thereby allowing the folded branch tube to move radially outward in the duct, (iv) advancing the branch tube and the branch sheath over the second guidewire towards and at least partially through the perforated duct wall, (v) removing the branch sheath from the branch tube, and (vi) removing the secondary sheath from the main tube in the duct (e.g., by moving the secondary sheath in the proximal or distal direction), thereby allowing the main tube to expand. The steps may be performed in the stated order.

In step (vi), due to the guidance provided by the second guidewire, the branch tube may be deployed from the main tube in a radially outward direction towards the anastomosis site. The tapered distal end of the branch sheath may be helpful for dilating the perforation in the duct wall while the additional stiffness of the branch sheath may be helpful for avoiding kinking.

In step (vi), the branch sheath and branch tube may be advanced along the second guidewire by advancing the delivery catheter and/or the main tube along the first guidewire in the distal direction or retracting the delivery catheter and/or the main tube along the first guidewire in the proximal direction. According to a presently preferred configuration, the branch sheath and branch tube are advanced along the second guidewire by advancing the delivery catheter and/or the main tube along the first guidewire in the distal direction. For this purpose, it is advantageous for the second end of the branch tube to be arranged distally of the first end.

As described above, removing the branch sheath from the branch tube in step (v) may comprise pulling the branch sheath off the branch tube via forceps. Such forceps may also be used for pulling the branch tube further through the duct wall and/or for grasping the branch tube in order to hold it in position while manipulating the delivery catheter and/or the main tube. The branch tube may be pulled further through the duct wall during step (v) but also thereafter.

According to a second aspect, the present invention relates to a method for minimally invasive creation of an anastomosis to a duct in a body of a patient with any of the afore-mentioned systems. The systems may be used in such method as described above.

The systems and methods according to the invention may be employed in various interventions, in particular in cardiothoracic and vascular surgery. For example, a ventricular assist device may be connected to the central circulation via the branch tube, e.g. by connecting one or more outflow cannulas of such ventricular assist device to the branch tube. As another example, the branch tube may be employed for creating an anastomosis in aortic arch surgery. As a future use, it is also envisaged to use the branch tube for connecting an artificial lung.

### Brief Description of the Drawings

For a better understanding, the invention will be described, by way of example only, with reference to the accompanying drawings in the following. The drawings are of exemplary nature only and are not intended to limit the scope of the present disclosure. The figures show:
- Fig. 1: a schematic illustration of a percutaneous transluminal insertion of a system according to the present invention into the thoracic aorta;
- Fig. 2: a schematic illustration of a system according to a first exemplary embodiment, the system comprising a sheath and a stent graft with an inverted branch tube;
- Fig. 3: a detail of Fig. 2;
- Fig. 4: a further detail of Fig. 2;
- Fig. 5: a schematic illustration of the system of Fig. 1 while being advanced to a target region of a duct over a first guidewire;
- Fig. 6: a schematic illustration of the system of Fig. 1 while the stent graft is being released;
- Fig. 7: a schematic illustration of the system of Fig. 1 while a wall of the duct is being perforated;
- Fig. 8: a detail of Fig. 7;
- Fig. 9: a schematic illustration of the system of Fig. 1 with a dilator inserted to an advanced position;
- Fig. 10: a schematic illustration of the system of Fig. 1 with the dilator being retracted in the proximal direction;
- Fig. 11: a schematic illustration of the system of Fig. 1 showing the attachment of a thread extending from a second end of the inverted branch tube to a proximal end of a second guidewire;
- Fig. 12: a schematic illustration of the system of Fig. 1 while the second guidewire is pulled through the perforated duct wall to pull the branch tube through the wall;
- Fig. 13: a schematic illustration of the system of Fig. 1 with the branch tube pulled through the wall to the outside of the duct;
- Fig. 14: a schematic illustration of a system according to a second exemplary embodiment, the system comprising a delivery catheter with a main tube and a branch tube;
- Fig. 15: a schematic illustration of first and second guidewires of the system of Fig. 14 advanced to the target region;
- Fig. 16: a schematic illustration of a steerable sheath being used to steer the second guidewire in order to perforate the duct wall of the duct;
- Fig. 17: a schematic illustration of the steerable sheath being retracted in the proximal direction;
- Fig. 18: a schematic illustration of the delivery catheter of Fig. 14 being advanced in the distal direction to the target region along the first and second guidewires;
- Fig. 19: a schematic illustration of the delivery catheter of Fig. 18 in the target region with a primary sheath being retracted;
- Fig. 20: a schematic illustration of the delivery catheter of Figs. 18 and 19, wherein the delivery catheter is further advanced in the distal direction to swing the branch tube with the branch sheath outwards, wherein at least the branch sheath passes through the perforated duct wall;
- Fig. 21: a schematic illustration of the delivery catheter of Figs. 18 to 20 with the branch sheath being removed from the branch tube;
- Fig. 22: a schematic illustration of the delivery catheter of Figs. 18 to 21 with the branch tube being held by forceps outside of the duct;
- Fig. 23: a schematic illustration of the delivery catheter of Figs. 18 to 22 showing a release of the main tube in the duct;
- Fig. 24: a schematic illustration of the delivery catheter of Figs. 18 to 23 showing the main duct in the fully expanded position, with the remaining delivery device being retracted in the proximal direction through the duct.

### Detailed Description

The terms "proximal" and "distal" are employed herein as generally used in the art. In particular, terms such as "distal direction" and "distally" refer to a direction away from the user of the system (i.e. the physician or surgeon). In other words, "distal direction" and "distally" refer to a direction away from the site where a respective element is percutaneously inserted into the body. Conversely, terms such as "proximal direction" and "proximally" refer to a direction towards the user of the system (i.e. the physician or surgeon) or towards the insertion site. In accordance therewith, elements, devices and tools are "advanced" into the body in the distal direction and "retracted" in the proximal direction.

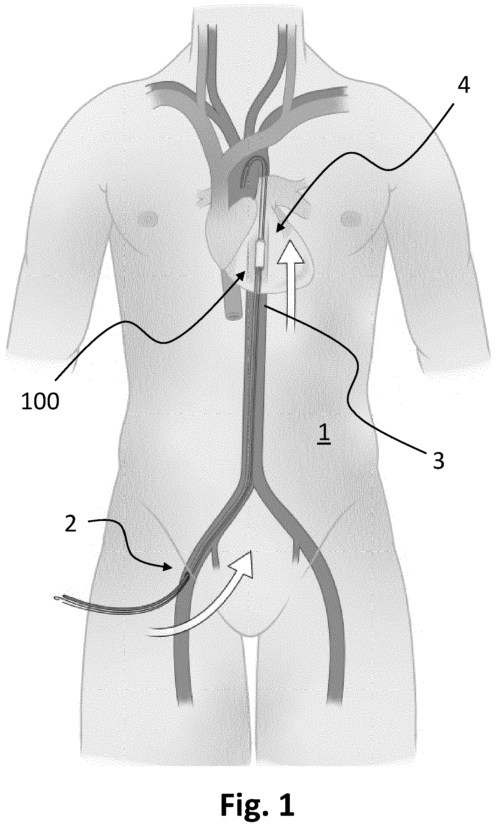

Fig. 1 schematically illustrates a percutaneous transluminal insertion of a system 100 according to the present invention into a body 1 of a patient at an insertion site 2. The system 100 is advanced through a duct to a target region 4. As shown in Fig. 1 in an exemplary manner, the duct may be a thoracic artery. However, other ducts (in particular other arteries and veins) may be treated. As further shown in Fig. 1, insertion may be provided through a femoral artery. However, as known in the art, other sites may be selected.

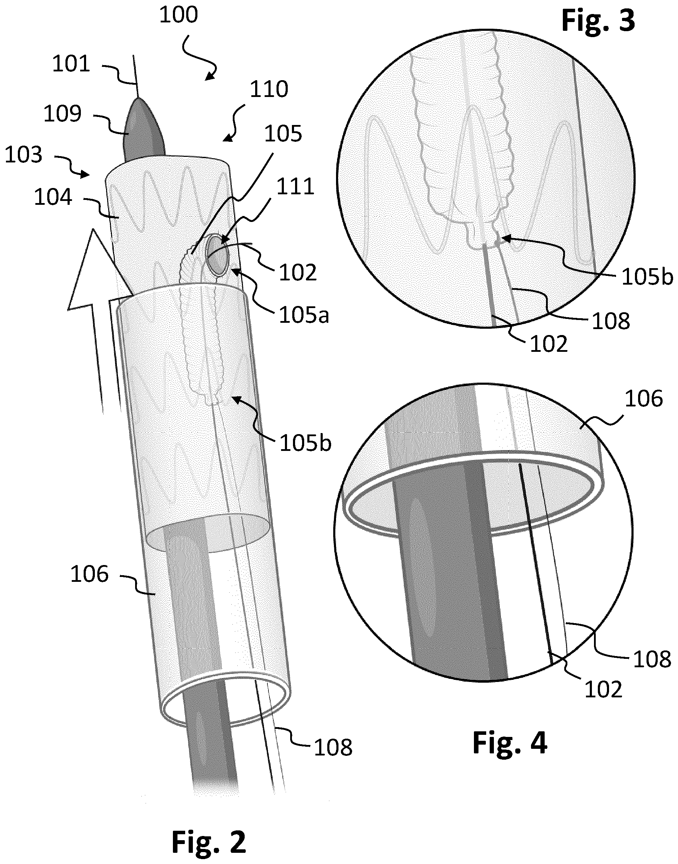

Figs. 2 to 13 illustrate the creation of an anastomosis with a system 100 according to a first exemplary embodiment of the present invention.

The system 100 comprises a first guidewire 101 and a second guidewire 102.

The system 100 further comprises a delivery catheter 110. In the delivery catheter 110, a stent graft 103 with a main tube 104 and a branch tube 105 is provided. The branch tube 105 has a first end 105a that is attached to a peripheral wall of the main tube 104 and a second end 105b opposite the first end 105a. The second guidewire 102 may extend into and/or through the branch tube 105. Preferably, the delivery device 110 is pre-fabricated and/or sold with the second guidewire 102 arranged in and/or extending through the branch tube 105.

The delivery catheter 110 further comprises a primary sheath 106 that is used to maintain the stent graft 103 in a compressed configuration (Fig. 1) in the delivery catheter 110. In this compressed configuration, the branch tube 105 is inverted and extends into the main tube 104. In particular, the second end 105b may be arranged in the main tube 104, as shown in Fig. 1.

The delivery catheter 110 may further include a shaft 109 on which the stent graft 103 and/or the primary sheath 106 may be supported. The shaft 109 may comprise an inner lumen (not shown) through which the first guidewire 101 extends. The shaft 109 may be provided with a tapered distal end in order to facilitate navigation thereof through the duct 3. The shaft 109 may extend through the stent graft 103 and/or the sheath 106. As such, the shaft 109 may also be referred to as an inner shaft 109.

A fenestration 111 is provided through the peripheral wall of the main tube 104 at the location where the first end 105a is attached.

As shown in detail in Figs. 3 and 4, a flexible wire or thread 108 is attached to the branch tube 105, in particular at the second end 105b. The flexible wire or thread 108 may extend proximally from the second end 105b through the primary sheath 106 and/or along the shaft 109.

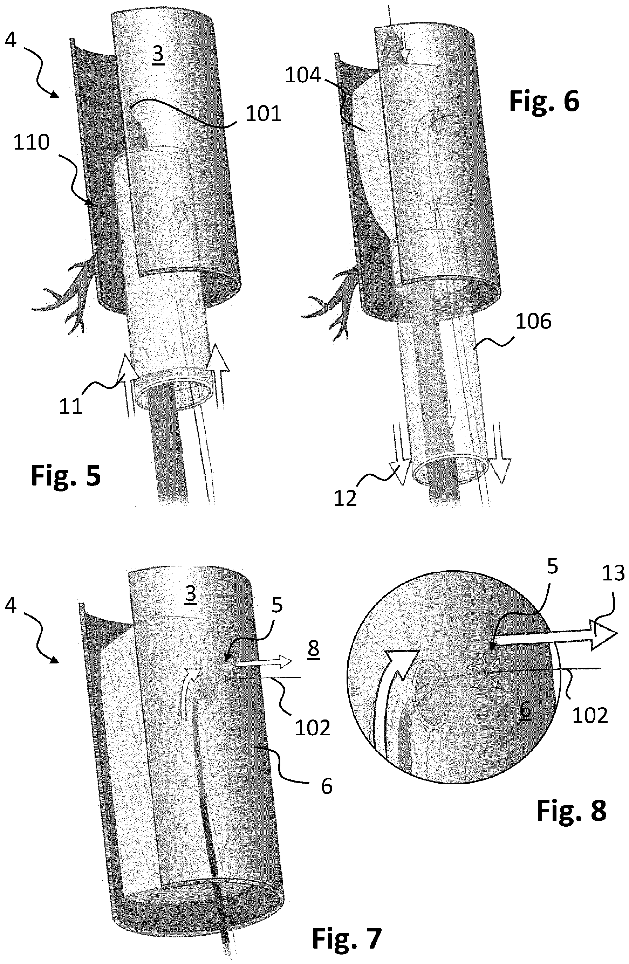

Fig. 5 schematically illustrates the delivery catheter 110 being advanced in the distal direction (as indicated by arrows 11) along the first guidewire 101 to the target region 4 in the duct 3. As shown, the second guidewire 102, which may be pre-arranged in the delivery device 110, may be advanced together with the delivery device 110 to the target region 4. The stent graft 103, including its main tube 104, are compressed by the primary sheath 106.

However, it is also envisaged to advance the second guidewire 102 to the target region before advancing the delivery catheter 110 to the target region over the first guidewire 101. The delivery catheter 110 may be advanced simultaneously over the first and second guidewires 101, 102 in this case.

Once the delivery catheter 110 has been arranged in the target region 4 as desired, the primary sheath 106 may be actuated in order to at least partially or fully expand the main tube 104 in the duct and/or at least partially or fully releasing the main tube 104 from the remaining delivery device 110 (see Fig. 6). Partially or fully releasing the main tube 104 may allow fixing the intended position of the stent graft 103 in the duct 3.

For this purpose, the primary sheath 106 may be retracted in the proximal direction, as indicated by arrows 12 in Fig. 6. Retracting the primary sheath 106 in the proximal direction is believed to be advantageous because the risk of colliding with the inverted branch tube 105, the thread 108 and/or the second guidewire 102 is reduced. However, also other release mechanisms are possible, as known for devices used in the release of self-expanding stents. Figs. 7 and 8 schematically illustrate a perforation through a wall 6 of the duct 3 at an anastomosis site 5 after the main tube 104 has been at least partially expanded.

As shown in Figs. 7 and 8, the perforation may be provided by extending the second guidewire 102 through the wall 6. For this purpose, the second guidewire 102 may be provided with an electrocautery tip at its distal end. The skilled person will appreciate that other ways of perforating the wall 6 are also feasible, such as the use of a guidewire with a sharp distal end or the use of a separate perforation device (such as a separate electrocautery device) that may be inserted over the second guidewire 102.

While Figs. 6 to 8 illustrate the perforation of the duct wall 6 after the main tube 104 has been at least partially expanded, this process could also be inversed. In other words, it is also feasible to perforate the duct wall 6 before at least partially expanding the main tube 104. The sequence shown in Figs. 6 to 8 is believed to be advantageous because it ensures that the perforation is performed at the location where the branch tube 105 is subsequently everted. The main tube 104 may be abutted against the wall 6 before performing the perforation at least in the region of the fenestration 111.

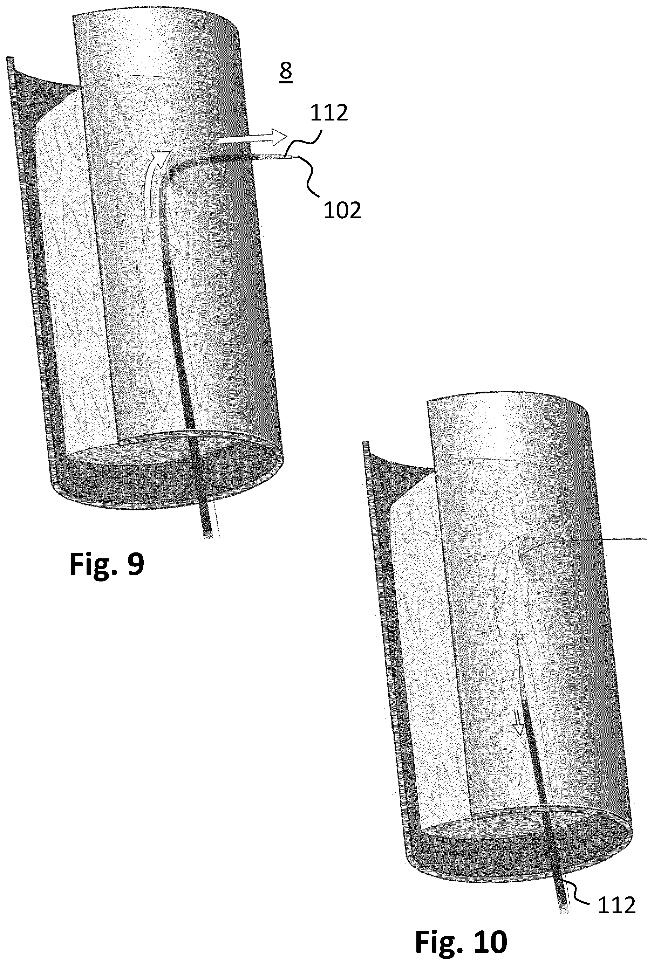

As further shown in Fig. 9, a dilator 112 may subsequently be advanced over the second guidewire 102 through the wall 6 at the anastomosis site 5 in order to dilate the perforation. The dilator 112 may subsequently be retracted in the proximal direction, as shown in Fig. 10. The dilator 112 may then be removed from the delivery device 110.

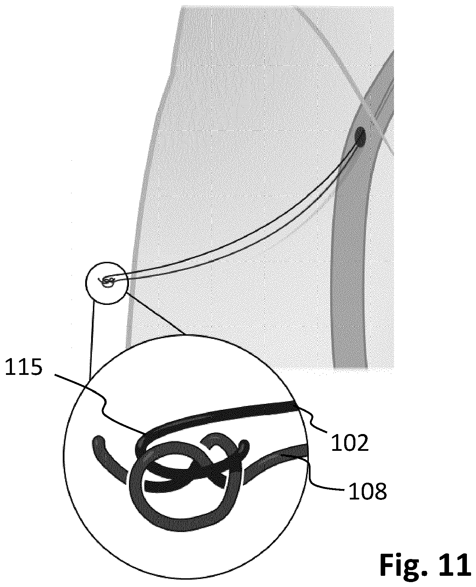

Figs. 11 to 13 schematically illustrate an exemplary procedure for everting the inverted branch tube 105.

As best shown in Fig. 11, a proximal end of the flexible wire or thread 108 may be coupled to the second guidewire 102. For example, the proximal end of the flexible wire or thread 108 and a proximal end of the second guidewire 102 may remain outside the body 1 of the patient when the delivery catheter 110 is advanced to the target region 4. The proximal end of the flexible wire or thread 108 may then be coupled to the proximal end of the second guidewire 102 by, for example, forming a knot. The proximal end of the second guidewire 102 may be provided with a hook 115 to facilitate the attachment.

The skilled reader will appreciate, however, that such coupling between the flexible wire or thread 108 and the second guidewire 102 may be provided in various ways. For example, the flexible wire or thread 108 could be attached to a portion of the second guidewire 102 closer to the second end 105b of the branch tube 105, e.g. a portion of the second guidewire 102 that is introduced into the body 1 of the patient when the delivery catheter 110 is advanced to the target region 4. Such attachment may be provided, in particular, during manufacture of the device. Moreover, it will be understood that other attachments than a knot may be used, such as an adhesive. Such various connections could be provided during an intervention (as described above) but also earlier, e.g. during pre-operative preparation or manufacture of the delivery catheter 110.

Furthermore, a temporary connection of the second end 105b of the branch tube 105 directly to the second guidewire 102 (i.e., without employing a flexible wire or thread 108) is envisaged.

For example, the second end 105b may be secured to the second guidewire 105b since the branch tube 105, despite such attachment, may allow for enough movement of the second guidewire 102 in order to perforate the wall 6. The second guidewire 102 could then be released by cutting off the second end 105b of the branch tube 105 after eversion of the branch tube 105. Alternatively, the connection could be such that it can be uncoupled in the body (e.g., a mechanical or adhesive connection that can be released may be employed).

As a further example, the second guidewire 102 may be provided with an abutment portion (not shown) that has an increased thickness. The abutment portion may be configured to engage with the branch tube 105 (e.g., with the second end 105b) when the second guidewire 102 is advanced through the wall 6. The second guidewire 102 would then drag along and evert the branch tube 105 when being moved further in the distal direction through the wall 6. The engagement may be released once the branch tube 105 has been sufficiently advanced through the wall 6, e.g. by loosening a constriction at the second end 105b, cutting off the second end 105b, or configuring the engagement such that it releases when a predetermined force is reached.

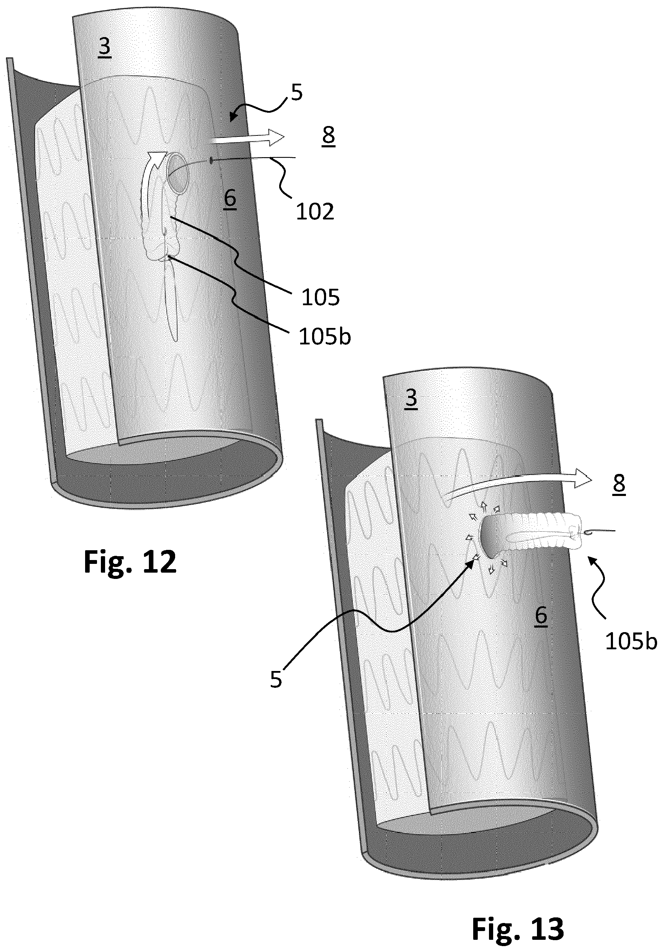

As further shown in Figs. 12 and 13, the second guidewire 102 may be advanced in the distal direction and may be pulled through the branch tube 105 and through the perforated duct wall 6 at the anastomosis site 5. The second end 105b is thus pulled through the branch tube 105, thereby everting the branch tube 105 and pulling it through the wall 6. Pulling the branch tube 105 through the wall 6 may dilate the perforation in the wall 6, as schematically depicted by the arrows shown in Fig. 13.

Once the branch tube 105 has been everted and pulled through the wall 6, the second guidewire may be disengaged from the branch tube 105. For example, the thread 108 may be cut, e.g., proximate the second end 105b.

As will be appreciated, the branch tube 105 is everted from an inverted position in which the second end 105b is arranged in the main tube 104 (see, e.g., Fig. 12) to an everted position in which the second end 105b is arranged on the outside 8 of the duct 3 (see Fig. 13).

When a flexible wire is used instead of the thread or the branch tube 105 is attached directly to the second guidewire 102, the wire may be mechanically disengaged from the second end 105b.

Alternatively, the branch tube 105 may be configured such that the second end 105b may be cut off.

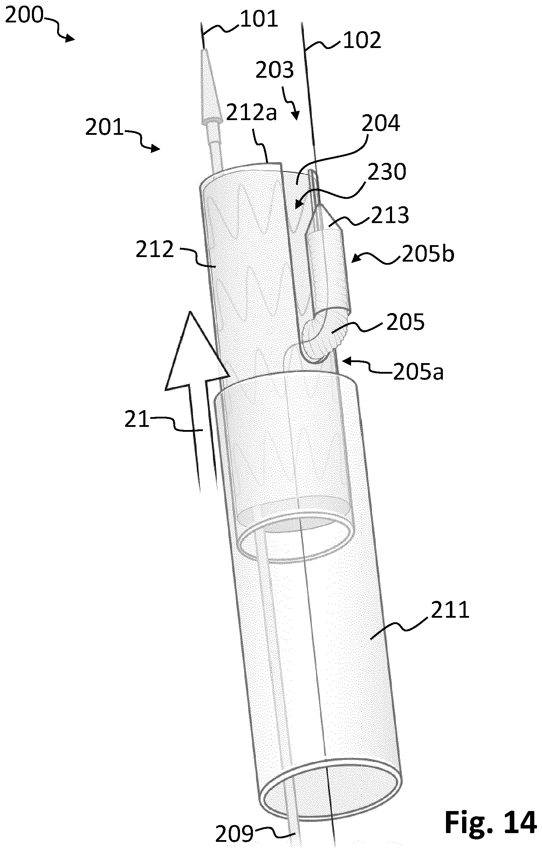

Figs. 14 to 24 illustrate the creation of an anastomosis with a system 200 according to a second exemplary embodiment of the present invention.

As shown in Fig. 14, the system 200 comprises a first guidewire 101, a second guidewire 102 and a delivery catheter 201. The delivery catheter 201 may comprise an inner shaft 209, which may support further components of the delivery catheter 201. The first guidewire 101 may run through the lumen of the inner shaft 209.

The delivery catheter includes a primary sheath 211 configured to extend around and overlap in the longitudinal direction with a main tube 204 and a branch tube 205 of a stent graft 203 arranged in a compressed configuration in the delivery catheter 201. In particular, the primary sheath is configured to maintain the branch tube 205 compressed and/or folded against the main tube 204 during advancement of the catheter 201 to the target region 4 through the duct 3. The primary sheath 211 may be overlapped with the main tube 204 and the branch tube 205 by moving it in the distal direction (see arrow 21) or (not shown) retracting it in the proximal direction.

A first end 205a of the branch tube 205 is attached to the peripheral wall of the main tube 204. Preferably, a second end 205b of the branch tube that is opposite to the first end 205a is located distally of the first end 205a when the branch tube is in the folded configuration, as illustrated in Fig. 14.

The delivery catheter 201 further comprises a secondary sheath 212. The secondary sheath 212 is configured to extend around and overlap in the longitudinal direction with the main tube 204. The secondary sheath 212 is configured to maintain the main tube 204 in the compressed configuration within the delivery catheter 201.

The secondary sheath 212 comprises an opening 230 through which the branch tube 205 extends out of the secondary sheath 212. The opening 230 extends to the distal end 212a of the secondary sheath 212.

The delivery catheter 201 further comprises a branch sheath 213. The branch sheath 213 is configured to extend around and overlap in the longitudinal direction with the branch tube 205. The branch sheath 213 may be configured to maintain the branch tube 205 in a compressed configuration.

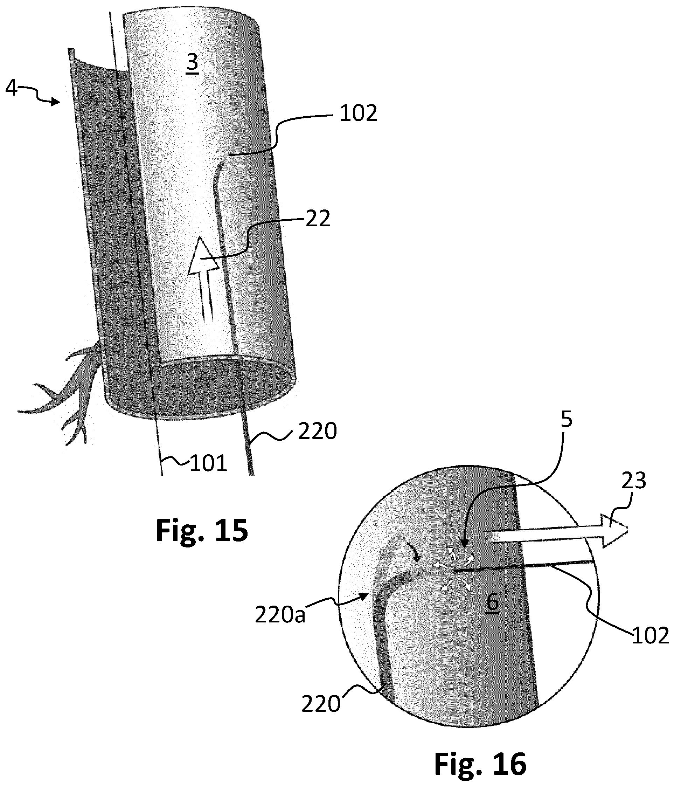

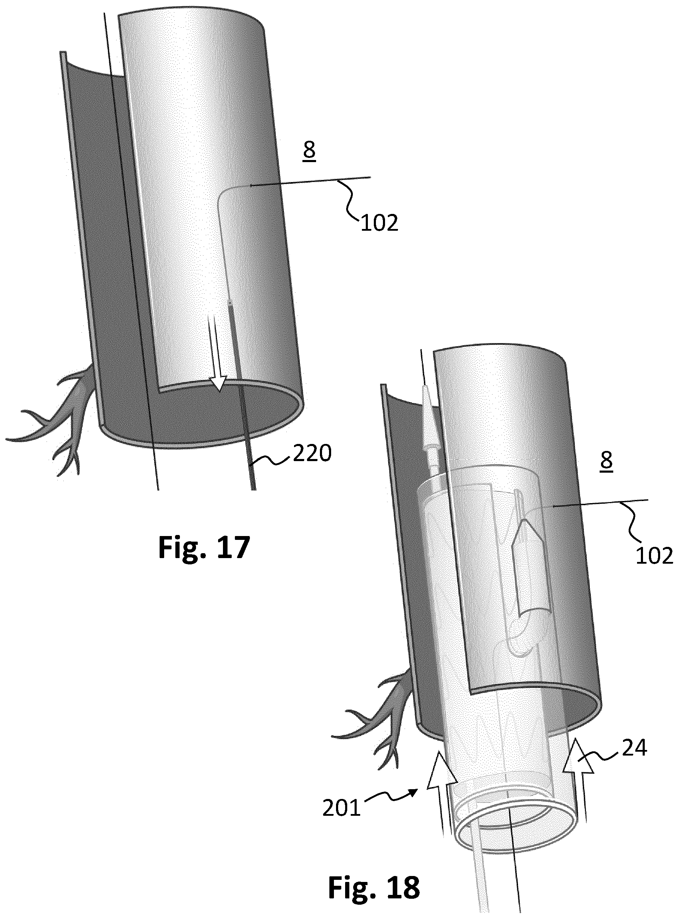

Figs. 15 to 17 show the first guidewire 101 and the second guidewire 102 advanced to the target region 4 of the duct 3. As shown, a steering sheath 220 may be advanced to the target region 4 over the second guidewire 102. The steering sheath 220 may have a steerable end 220a that may be used to position a distal end of the second guidewire 102 in order to perforate the wall 6 of the duct 3 at the desired anastomosis site 5 (see Fig. 16). The steering sheath 220 may subsequently be retracted along the second guidewire 102 (see Fig. 17).

With the first guidewire 101 and the second guidewire 102 in place, the delivery catheter 201 may be advanced in the distal direction (see arrows 24) to the target region 4, as shown in Fig. 18. This may be referred to as a simultaneous advancement of the delivery catheter 201 over the first and second guidewires 101, 102.

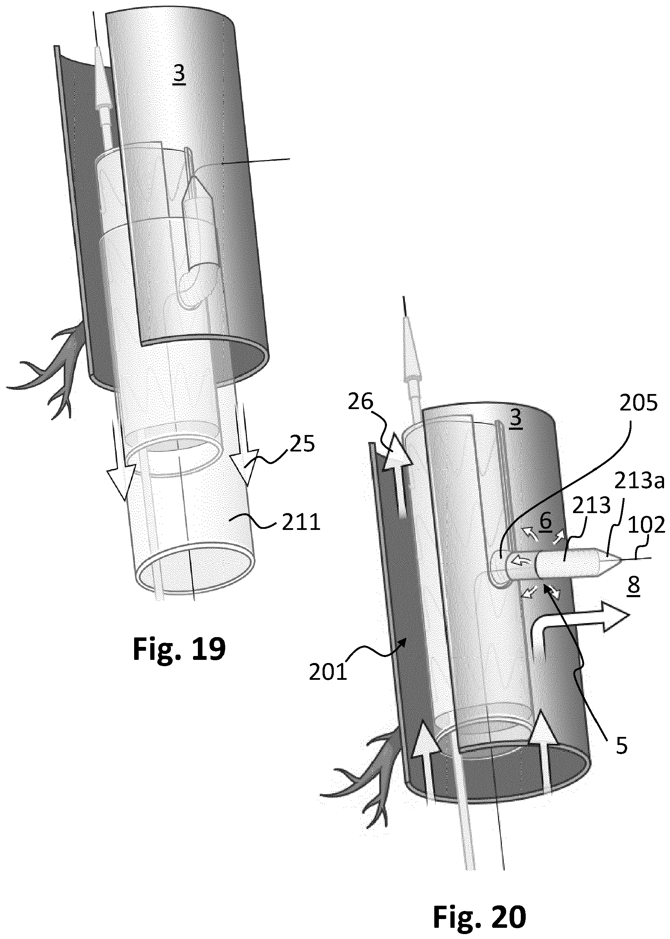

Once the delivery catheter 201 is positioned in the target region 4, the primary sheath 211 may be moved to release the branch tube 205 (see Fig. 19), for example by retracting the primary sheath 211 in the proximal direction (as indicated by arrows 25).

As best shown in Fig. 20, the delivery device 201 may then be moved further in the distal direction while the second guidewire 102 remains in position inserted into and/or extending through the wall 6 of the duct 3. Since the second guidewire 202 extends through the branch tube 205 and the branch sheath 213, the branch tube 205 and the branch sheath 213 are advanced along the second guidewire 102 during such movement. The branch tube 205 thus unfolds and/or swings in a radially outward direction from its folded configuration as shown in Fig. 19. The branch sheath 213 covering the branch tube 205 is pushed against the inner periphery of the wall 6 at the anastomosis site 5 and ultimately through the wall 6, thereby leading the branch tube 205 through said wall (see Fig. 20).

The insertion of the branch sheath 213 through the wall 6 may be facilitated by providing the branch sheath 213 with a tapering (e.g. conical) distal end 213a that dilates the perforation provided by the second guidewire 102 at the anastomosis site 5.

The insertion of the branch sheath 213 through the wall 6 may also be facilitated by providing the branch sheath 213 with an increased stiffness and/or rigidity, in particular a higher stiffness and/or rigidity than the branch tube 205, more specifically a higher stiffness and/or rigidity than the branch tube 205 against radial compression and/or against buckling in the longitudinal direction.

While the second guidewire 102 is shown to extend through the branch tube 205 and through the branch sheath 213 in the exemplary embodiment illustrated in Fig. 20, it will be appreciated that the second guidewire 102 may guide these elements to the anastomosis site 5 in various ways. For example, the second guidewire 102 could extend only through the branch sheath 213 and not through the branch tube 205. The branch sheath 213 could be provided, for example, with a separate lumen (not shown) or one or more eyelets (not shown) for receiving the second guidewire 102 therethrough. The invention, therefore, is not limited to the configuration illustrated in Figs. 14 to 24.

While a movement of the delivery catheter 201 in the distal direction is shown in order to unfold the branch tube 205 radially outward, it should be noted that a movement in the proximal direction may also be envisaged, in particular if the second end 205b of the branch tube 205 is located proximally from the first end 205a in the folded configuration (not shown). Without wanting to be bound by theory, it is presently believed, however, that this may require a more sophisticated routing of the second guidewire (i.e., partially in the proximal direction).

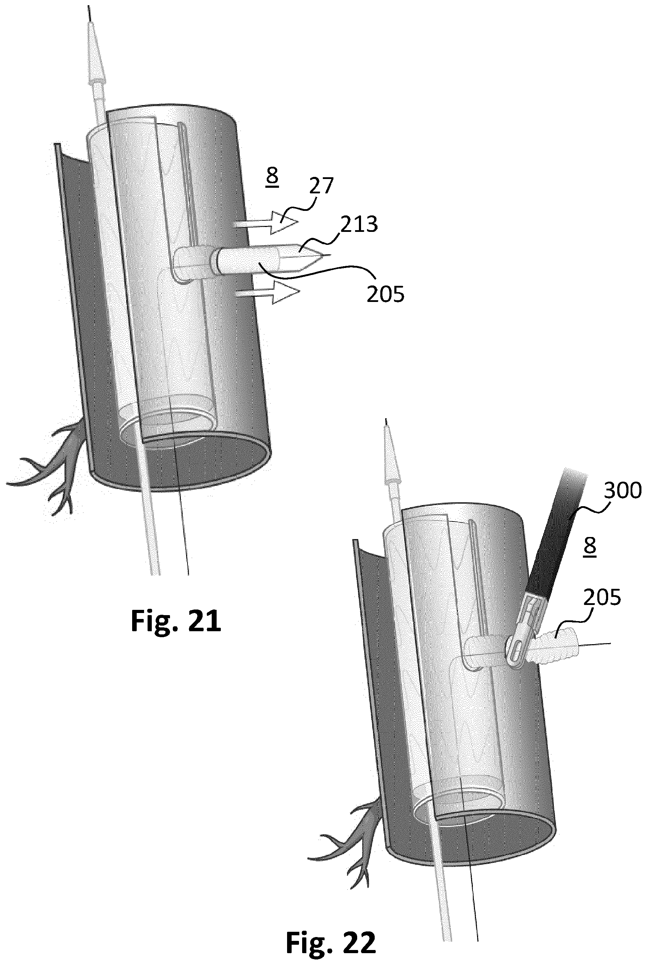

As illustrated in Fig. 21, the branch sheath 213 may subsequently be removed from the branch tube 205 by moving the branch sheath 213 entirely through the wall 6 of the duct 3. Various mechanisms and procedures may be relied upon for this purpose. For example, the branch sheath 213 may be pulled through the wall 6 by forceps inserted into the body 1 from the outside 8 the duct 3 (cf. forceps 300 in Fig. 22; not shown in Fig. 21). The forceps may then be used for removing the branch sheath 213 from the body 1. To facilitate removal from the remaining catheter 201, the branch sheath 213 and the secondary sheath 212 may be provided as separate elements.

After removing the branch sheath 213, the branch tube 205 may be grasped with forceps 300 inserted into the body 1 from the outside 8 of the duct 3. The position of the branch tube 205 may be modified in this manner and/or the position of the branch tube 205 may be secured until the position of the main tube 204 in the duct 3 is fixated.

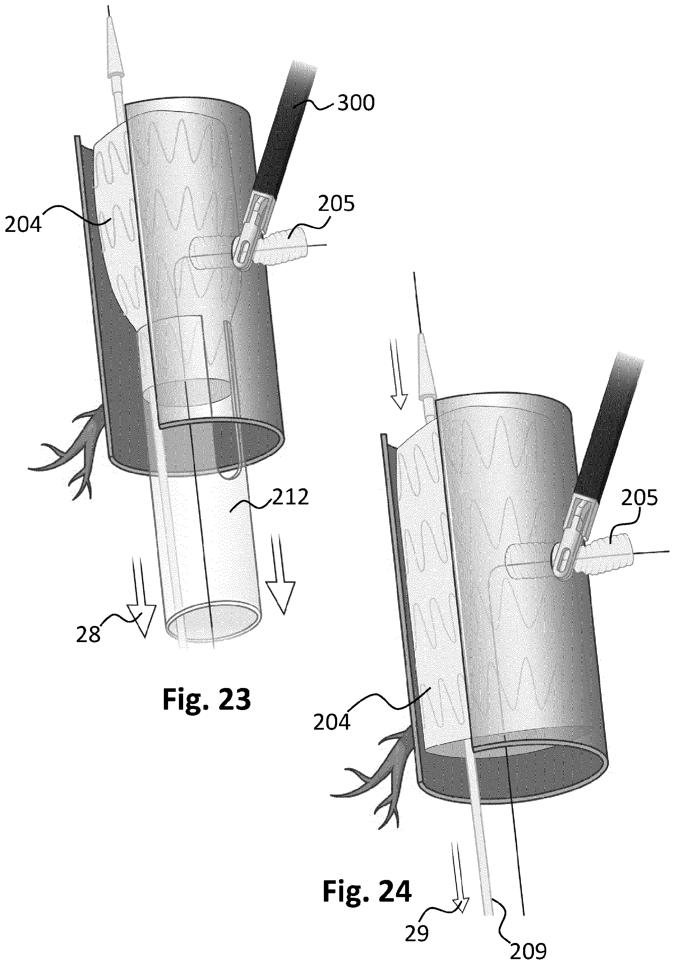

Fig. 23 shows the secondary sheath 212 being moved in the proximal direction (see arrow 28) in order to release and expand the main tube 204 in the duct 3. Subsequently (see Fig. 24), the inner shaft 209 may be removed.

Although the present invention has been described to a certain degree of particularity, it should be understood that various alterations and modifications could be made without departing from the spirit or scope of the invention as hereinafter claimed.

For example, while it is believed that the systems and methods described and claimed herein are particularly advantageous for providing an anastomosis through an artificially created perforation in the wall of the duct, it will be appreciated that the systems and methods disclosed herein could also be employed for providing an anastomosis where no artificial perforation is required, e.g. where the duct comprises a natural branching (e.g., if a side branch of the duct extending from a main duct at the branching is to be anastomosed and/or replaced). Therefore, perforation of the duct wall is not necessarily required.

## Claims

1. A system (100, 200) for minimally invasive creation of an anastomosis to a duct (3) in a body (1) of a patient, wherein the anastomosis is created in a target region (4) of the duct (3) at an anastomosis site (5), wherein the duct (3) comprises a duct wall (6), and wherein said duct wall (6) is perforated at the anastomosis site (5), the system (100, 200) comprising:
a delivery catheter (110, 201) configured for percutaneous transluminal insertion through the duct (3) to the target region (4), wherein the delivery catheter (110, 201) is configured to be guided to the target region (4) via a first guidewire (101);
a stent graft (103, 203) arranged within the delivery catheter (110, 201), the stent graft (103, 203) comprising a main tube (104, 204) and at least one branch tube (105, 205), wherein the branch tube (105, 205) is configured to be guided to the anastomosis site (5) via a second guidewire (102);
wherein the main tube (104, 204) is configured to be expanded in the duct (3);
wherein the branch tube (105, 205) extends from a first end (105a, 205a) to a second end (105b, 205b), the first end (105a, 205a) being coupled to the main tube (104, 204) and the second end (105b, 205b) being configured for insertion through the punctured duct wall (6) at the anastomosis site (5).

2. The system (100, 200) of claim 1, wherein, the branch tube (105, 205) is configured for insertion through the punctured duct wall (6) from within the duct (3) towards outside (8) the duct (3).

3. The system (100, 200) of claim 1 or 2, further including a primary sheath (106, 211) extending around the main tube (104, 204) and the branch tube (105, 205).

4. The system (100) according to any one of the preceding claims, wherein the stent graft (103) is arranged within the delivery catheter (110) with the branch tube (105) in an inverted position, wherein the second end (105b) of the branch tube (106) is located in the main tube (104) in said inverted position.

5. The system (100) of claim 4, wherein the system (100) is configured for everting the branch tube (105) from the inverted position to an everted position, wherein the second end (105b) of the branch tube (105) is located outside the main tube (104) in said everted position.

6. The system (100) of claim 4 or 5, wherein the system (100) is configured for inserting the branch tube (105) through the duct wall (6) by everting the branch tube (105) from the inverted position to the everted position.

7. The system (100) of claim 4, 5 or 6, further comprising a flexible wire or thread (108) coupled to the branch tube (105), preferably to the second end (105b) of the branch tube (105).

8. The system (100) of claim 7, wherein the flexible wire or thread (108) is configured to be coupled with the second guidewire (102).

9. The system (100) of claim 7 or 8, wherein the branch tube (105) is everted by pulling the flexible wire or thread (108) through the duct wall (6) at the anastomosis site (5).

10. The system (200) according to any one of claims 1 to 3, wherein the stent graft (203) is arranged within the delivery catheter (201) with the second end (205b) of the branch tube (205) located outside the main tube (204), wherein the delivery device (201) further comprises at least one branch sheath (213) arranged over the branch tube (205).

11. The system (200) of claim 10, wherein the branch sheath (213) is configured for being at least partially or entirely inserted through the duct wall (6).

12. The system (200) of claim 10 or 11, wherein the branch sheath (213) is configured to be removed from the branch tube (205) after guiding and/or at least partially inserting the branch tube through the duct wall (6).

13. The system (200) of claim 10, 11 or 12, wherein branch sheath (213) is stiffer than the branch tube (205).

14. The system (200) according to any one of claims 10 to 13, wherein the branch sheath (213) comprises a tapered distal end, preferably a conical distal end.

15. The system (200) according to any one of claims 10 to 14, wherein the delivery catheter (110) further comprises a secondary sheath (212), wherein the secondary sheath (212) extends around the main tube (204) and comprises an opening (230), wherein the branch tube (205) extends through said opening (204), preferably wherein the opening (204) extends up to a distal end (212a) or up to a proximal end of the secondary sheath (212).
